(19) Europäisches Patentamt
European Patent Office
Office européen des brevets

(11) Numéro de publication: **0 000 299**
**B1**

(12) # FASCICULE DE BREVET EUROPEEN

(21) Numéro de dépôt: **78400009.3**

(22) Date de dépôt: **01.06.78**

(51) Int. Cl.³: **C 07 C 103/50,**
C 07 D 295/14,
C 07 D 233/60,
A 61 K 31/16,
A 61 K 31/395

(54) **Nouveaux dérivés de benzoyl-2-chloro-4-glycinanilides substitués, leur procédé de préparation et leur application en tant que médicaments**

(30) Priorité: **16.06.77 FR 7718511**

(43) Date de publication de la demande:
**10.01.79 Bulletin 79/01**

(45) Mention de la délivrance du brevet:
**12.11.80 Bulletin 80/23**

(84) Etats contractants désignés:
**BE CH DE FR GB NL**

(56) Documents cités:
**FR - A - 2 012 022**
**FR - A - 2 021 010**
**FR - A - 2 244 482**
**FR - M - 1 397**

(73) Titulaire: **PIERRE FABRE S.A.**
**125, rue de la Faisanderie**
**F—75116 Paris (FR)**

(72) Inventeur: **Mouzin, Gilbert**
**21, rue Sainte Foy**
**F—81100 Castres (FR)**
**Cousse, Henri**
**Foun de las Nibios Chemin de Lastinos**
**F—81100 Castres (FR)**
**Stenger, Antoine**
**11, rue des Cigales**
**F—81100 Castres (FR)**
**Casadio, Sylvano**
**Via Tantardini 15**
**I—20136 Milan (IT)**

(74) Mandataire: **Corre, Jacques Denis Paul, et al**
**Cabinet Regimbeau 26, Avenue Kléber**
**F—75116 Paris (FR)**

**EP 0 000 299 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Nouveaux dérivés de benzoyl-2 chloro-4 glycynanilides substitués, leur procédé de préparation et leur application en tant que médicaments.

La présente invention réalisée au Centre de Recherches PIERRE FABRE concerne de nouveaux dérivés de benzoyl-2 chloro-4 glycinanilides substitués, leur procédé de préparation et leur application en tant que médicaments.

Dans la technique antérieure la plus proche, par exemple illustrée par le Brevet Spécial de Médicament français n° 1397 M, on a déjà proposé, à titre de médicaments doués de propriétés anti-convulsives et tranquillisantes, des dérivés de 2-amino-acétamidobenzophénones.

Il s'est avéré cependant que de tels composés à structure ouverte présentent une très grande instabilité et ont spontanément tendance à se cycliser pour donner naissance à des composés à structure benzodiazépinique fermée.

Les travaux de Von H. Oelschläger & Coll. (Arzneimittel Forsch. 1973, pages 802 et suivantes) ont ensuite révélé que les benzophénones à structure ouverte étaient inactives et que la présence d'un cycle diazépinique était la condition sine qua non pour obtenir une activité.

La présente invention se rapporte en revanche à des dérivés se distinguant, de ceux de la technique antérieure précitée, par une substitution sur le premier atome d'azote en série glycynanilidique, répondant à la formule:

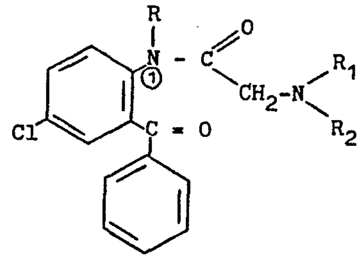

Or, il a été démontré que cette substitution en série glycynanilidique influençait la distance N—N, laquelle apparaît comme étant un paramètre déterminant pour l'activité de ce type de composés.

Il convient de noter que cette constatation est tout à fait surprenante, compte tenu du fait que dans le cas de composés benzodiazépiniques il n'existe qu'une faible différence d'activité entre le diazépam et le diméthlydiazépam, alors qu'en série glycynanilidique la différence d'activité s'est révélée très importante.

Les nouveaux dérivés de la présente invention repondent à la formule générale I

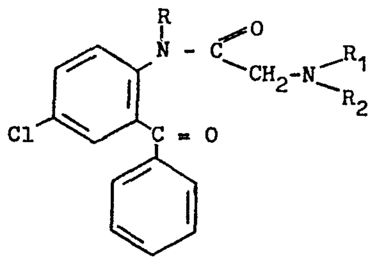

(I)

dans laquelle

R représente un groupe alcoyle linéaire ou ramifié, de préférence un groupe alcoyle inférieur;

$R_1$ et $R_2$ peuvent être identiques ou différents et sont choisis parmi l'hydrogène, les groupes alcoyle, alcényle, alcynyle, hydroxyalcoyle, alcoxyalcoyle, aralcoyle, ces divers groupes pouvant être linéaires ou ramifiés, et parmi les groupes cycloalcoyle ayant de 3 à 6 chaînons et étant éventuellement substitués sur le carbone en $\alpha$ par un radical alcynyle;

les groupes $R_1$ et $R_2$ pouvant en outre former avec l'atome d'azote auquel ils sont reliés un hétérocycle azoté à 5 ou 6 chaînons, saturé ou non, et comprenant éventuellement un second hétéroatome choise parmi l'oxygène et l'azote, ledit hétérocycle étant éventuellement substitué, de préférence sur le second atome d'azote, par un radical alcoyle inférieur;

ainsi que leurs sels obtenus avec des acides minéraux ou organiques thérapeutiquement acceptables.

On donnera ci-après une explication illustrative de quelques significations données à propos des radicaux R, $R_1$ et $R_2$. Les groupes alcoyle désignent des groupes alcoyle à chaîne linéaire ou ramifiée, tels que méthyle, éthyle, propyle, isopropyle, butyle, isobutyle, sec.butyle, tert.-butyle, pentyle, isopentyle, néopentyle, tert.pentyle, hexyle, isohexyle, heptyle, etc. Les substituants alcoxy peuvent par exemple être choisis parmi les groupes méthoxy, éthoxy, propoxy, isopropoxy, etc. Les groupes aralcoyle peuvent être choisis parmi les groupes benzyle, phénéthyle, phénylpropyle, etc. Les groupes alcényle peuvent être choisis parmi les groupes allyle, butényle, pentadiényle, etc. Les groupes alcynyle peuvent être choisis parmi les groupes éthynyle, propargyle, etc. Enfin, les hétérocycles saturés ou non

sont choisis par exemple parmi les groupes pyrrolyle, imidazolyle, pyrazolyle, isoxazolyle, pyridyle, pyrazinyle, pyrinidinyle, pyridazinyle, pyrrolidinyle, imidazolidinyle, pyrazolidinyle, pipéridyle, pipérazinyle, morpholinyle, etc.

La présente invention s'applique également aux sels des composés de formule I obtenus avec des acides thérapeutiquement acceptables. A titre d'exemples non limitatifs de sels d'addition thérapeutiquement ou physiologiquement acceptables, on peut citer les sels d'acides minéraux, tels que les acides chlorhydrique, phosphorique et sulfurique, et les sels d'acides organiques, tels que les acides succinique, tartrique, etc.

La présente invention concerne également un procédé de préparation des composés de formule I, consistant à faire réagir un composé de formule générale II

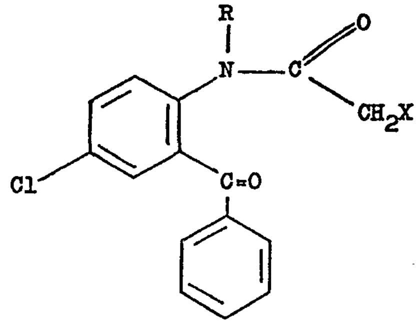

(II)

dans laquelle:

R a la signification donnée à propos de la formule générale I et X représente un atome d'halogène, avec une amine de formule générale III

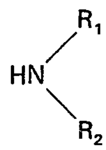

(III)

dans laquelle:

$R_1$ et $R_2$ ont la signification donnée à propos de la formule générale I.

Les produits de départ de formule générale II peuvent être préparés selon l'un des schémas réactionnels suivants:

a) *Procédé au tosylate*

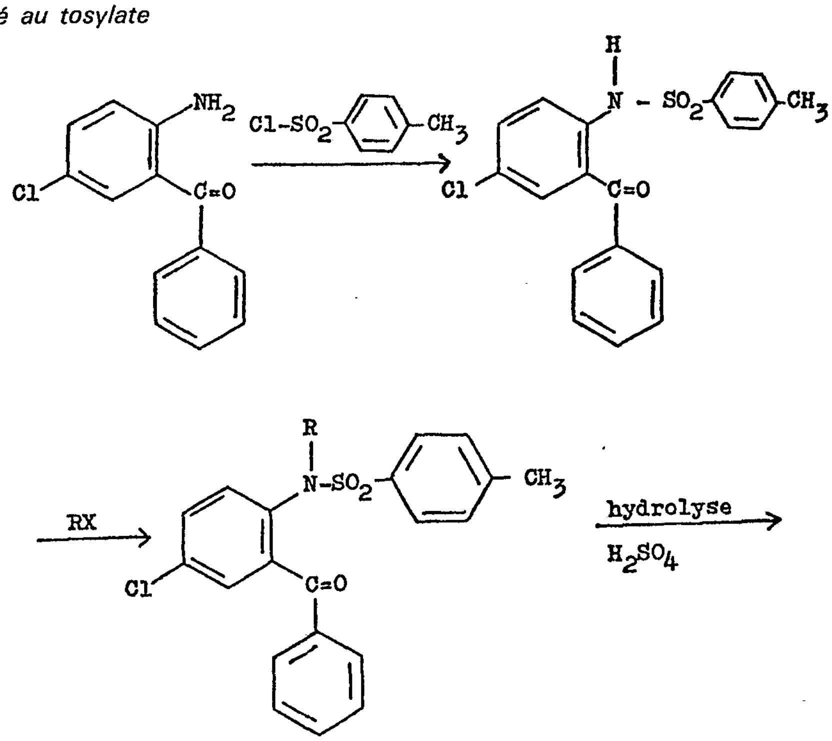

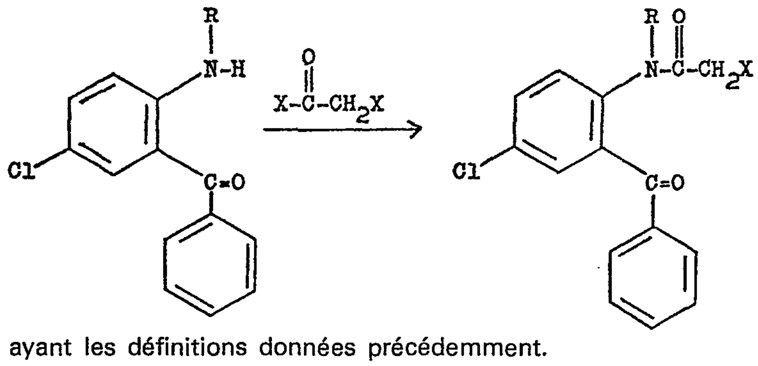

X et R ayant les définitions données précédemment.

b) *Procédé au sulfate d'alcoyle*

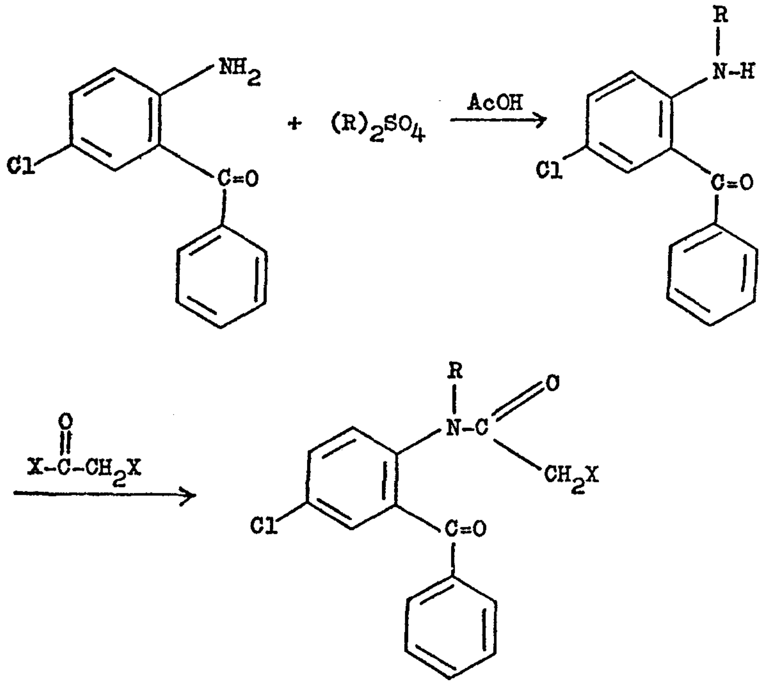


La présente invention concerne enfin l'application des composés de formule générale I en tant que médicaments doués d'une activité sur le système nerveux central et en particulier en tant qu'agents anxiolytiques, sédatifs, anticonvulsifs, hypnotiques ou comme agent de relaxation musculaire.

La présente invention sera décrite ci-après plus en détails à propos des exemples non limitatifs suivants:

Exemple 1

*Chlorohydrate de N-isopropyl, N' méthyl (benzoyl-2 chloro-4) glycynanilide*

a) Préparation de la tosylamido-2 chloro-5 benzophénone

A une solution de 208,5 g (0,9 mole) d'amino-2 chloro-5 benzophénone dans 500 $cm^3$ de pyridine, on ajoute 190,6 g de chlorure de tosyle de façon que la température du milieu réactionnel ne dépasse pas 48°C. Après cette addition on maintient l'agitation pendant 15 minutes, puis on porte à 100°C pendant 1 heure.

On obtient ainsi une solutin homogène de couleur brune. On laisse revenir à température ambiante, puis on hydrolyse le milieu réactionnel en le versant dans 4 litres d'acide chlorhydrique 3 N en présence de glace. On obtient alors une huile épaisse qui cristallise.

Les cristaux sont récupérés par filtration, puis on dissout ces cristaux dans deux litres d'acétate d'éthyle, on décante la phase aqueuse formée, séche la phase organique sur sulfate de sodium et on décolore par addition de noir animal. Après filtration, on évapore la phase organique. On récupère un résidu brut cristallisé que l'on triture dans l'ether de pétrole. La coloration disparaît en partie et l'on obtient après filtration et séchage 330 g de cristaux jaune-pâle. Rendement 95%.

Point de fusion: 123,5°C

Chromatographie sur plaque:

—support: gel de silice 60 F 254 Merck

—solvant: acétate d'éthyle—éther de pétrole 10/90
—révélation: UV et iode
—Rf: 0,26

b) Préparation de la N-méthyl tosylamido-2 chloro-5-benzophénone

On dissout 308,7 g (0,8 mole) de tosylamido-2 chloro-5 benzophénone dans deux litres de toluène, puis on additionne, en maintenant le milieu réactionnel à 20°C, une solution de 18,5 g (0,805 atome/g) de sodium dans 300 $cm^3$ de méthanol.

On maintient l'agitation 15 minutes puis on additionne goutte à goutte 201,8 g de sulfate de méthyle.

On agite à température ambiante pendant 6 heures et on laisse une nuit au repos, puis on porte à 50°C pendant 2 heures et à 70°C pendant 3 heures.

On ajoute un litre de soude 3 N et on agite pendant 1/2 heure. On décante, lave 3 fois à l'eau jusqu'à pH neutre, puis on sèche sur sulfate de sodium et on décolore au noir animal.

On filtre, évapore jusqu'à siccité, l'huile épaisse obtenue étant dissoute dans l'éthanol. On obtient, en glaçant, des cristaux que l'on recristallise dans l'éthanol.

On récupère après filtration et séchage 279,5 g de cristaux blancs. Rendement 87%.

Point de fusion: 105°C

Chromatographie sur plaque:
—support: gel de silice 60 F 254 Merck
—solvant: acétate d'éthyle—éther de pétrole 10/90
—révélation: UV et iode
—Rf: 0,24

c) Préparation de la méthyl-amino-2 chloro-5 benzophénone

Dans 600 g de glace, on ajoute lentement 1400 $cm^3$ d'acide sulfurique à 96%. On porte à 110°C puis on additionne 240 g (0,6 mole) de N-méthyl tosylamido-2 chloro-5 benzophénone, maintient 20 minutes à cette température et on laisse revenir à température ambiante.

On jette dans 6 litres d'eau glacée, la base libre, relargue et cristallise. On récupère les cristaux par filtration. On dissout ces cristaux dans 1,5 litre d'acétate d'éthyle, décante la phase aqueuse et sèche la phase organique sur sulfate de sodium. Après filtration et concentration on récupère 124,5 g de cristaux jaunes. Rendement 85%.

Point de fusion: 94°C

Chromatographie sur plaque:
—support: gel de silice 60 F 254 Merck
—solvant: acétate d'éthyle—éther de pétrole 10/90
—révélation: UV et iode
—Rf: 0,55

Remarque:

Le méthyl amino-2 chloro-5 benzophénone peut être également préparée par méthylation directe de l'amino-2 chloro-5 benzophénone selon le mode opératoire suivant:

A une suspension de 100 g d'amino-2 chloro-5 benzophénone dans 500 $cm^3$ d'acide acétique, on ajoute 42 $cm^3$ de sulfate de méthyle et on porte deux heures au reflux.

On hydrolyse par deux litres d'eau et extrait au chloroforme.

On sèche la phase organique sur sulfate de sodium.

Après évaporation de la phase organique, on récupère une huile qui cristallise lentement. Par recristallisation dans le méthanol on obtient 70,8 g de cristaux jaunes. Rendement: 86%.

Point de fusion: 93,5°C.

d) Préparation du N-méthyl (benzoyl-2 chloro-4) phényl bromacétamide

On glace à 0°C une solution de méthylamino-2 chloro-5 benzophenone dans un mélange de 200 $cm^3$ de benzène et 100 $cm^3$ d'éther et on ajoute goutte à goutte en 25 minutes 5,8 $cm^3$ de chlorure de bromacétyle en solution dans 40 $cm^3$ d'éther. On laisse une nuit sous agitation à température ambiante puis on évapore jusqu'à siccité. Le résidu huileux trituré dans l'éther de pétrole cristallise rapidement. On filtre, lave à l'éther de pétrole, puis dissout dans l'acétate d'éthyle et on décolore au noir animal. Après filtration et concentration, précipitation par l'éther de pétrole et filtration, on récupère 19,3 g de produit. Rendement: 82%.

Point de fusion: 90°C

Chromatographie sur plaque:
—support: gel de silice 60 F 254 Merck
—solvant: acétate d'éthyle—éther de pétrole 25/75
—révélation: UV et iode
—Rf: 0,43

e) Préparation du chlorhydrate de N-isopropy, N' méthyl (benzoyl-2 chloro-4) glycynanilide

A une solution de 34 $cm^3$ d'isopropylamine dans 200 $cm^3$ d'acétone, on ajoute par spatulées 16,98 g de N-méthyl (benzoyl-2 chloro-4) phényl bromacétamide. La dissolution est immédiate et assez exothermique.

Après addition du dérivé bromé, on chauffe 6 heures à 45°C puis on laisse une nuit au repos et on évapore jusqu'à siccité sous pression réduite. On reprend le résidu par une solution bicarbonatée et extrait à l'acétate d'éthyle. On lave à l'eau, sèche sur sulfate de sodium et décolore au noir animal. Après filtration, on évapore à sec. L'huile résiduelle est traitée par une solution éthanolique saturée d'acide chlorhydrique.

On ajoute de l'éther éthylique pour faciliter la cristallisation, on filtre et on sèche. On récuptère 13,18 g de produit de formule:

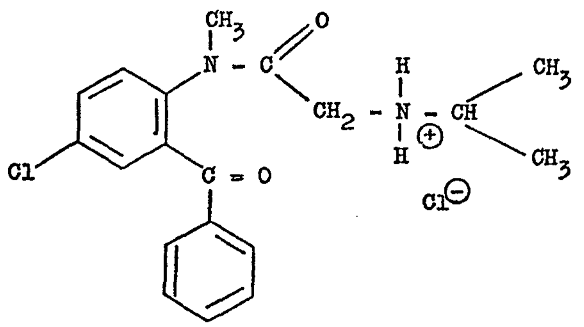

Formule brute: $C_{19}H_{22}Cl_2N_2O_2$
Masse moléculaire: 381,29
Cristaux blancs
Point de fusion: 239°C
Chromatographie sur plaque:
—support: gel de silice 60 F 254 Merck
—solvant: butanol—acide acétique—eau 6/2/2
—révélation: UV et iode
—Rf: 0,53
Solubilité: soluble dans l'eau à 1%.

Exemple 2

*Chlorhydrate de N-méthyl N'-diméthyl-1-1 propargyl (benzoyl-2 chloro-4) glycynanilide*

D'une manière analogue à celle de l'exemple 1, mais en utilisant la diméthyl-1-1 propargylamine, on obtient le produit de formule:

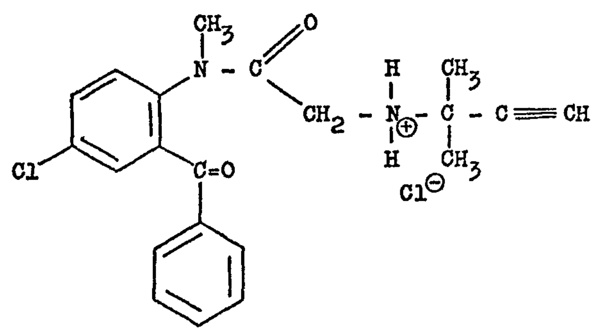

Formule brute: $C_{21}H_{22}Cl_2N_2O_2$
Masse moléculaire: 405,33
Cristaux blancs
Point de fusion: 185°—186°C
Chromatographie sur plaque:
—support: gel de silice 60 F 254 Merck
—solvant: acétate d'éthyle—éther de pétrole 50/50
—révélation: UV et iode
—Rf: 0,37
Solubilité: soluble dans l'eau à 1%.

Exemple 3

*Chlorhydrate de N-N' diméthyl (benzoyl-2 chloro-4) glycynanilide*

D'une manière analogue à celle de l'exemple 1, mais en utilisant la méthylamine on obtient le produit de formule:

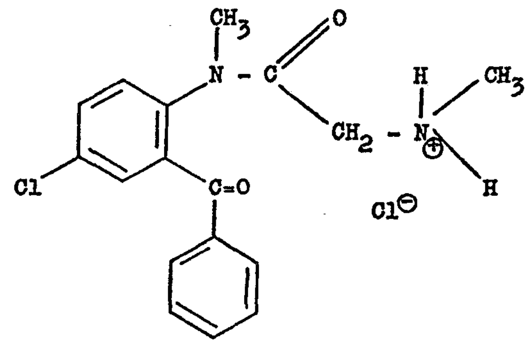

Formule brute: $C_{17}H_{18}Cl_2N_2O_2$
Masse moléculaire: 353,25
Cristaux blancs
Point de fusion: 180°C
Chromatographie sur plaque:
—support: gel de silice 60 F 254 Merck
—solvant: butanol—acide acétique—eau 6/2/2
—révélation: UV et iode
—Rf: 0,37
Solubilité: soluble dans l'eau à 15%

Exemple 4
*N,N'-N' triméthyl (benzoyl-2 chloro-4)-glycynanilide*

D'une manière analogue à celle de l'exemple 1, mais en utilisant la diméthyl amine, on obtient le produit de formule:

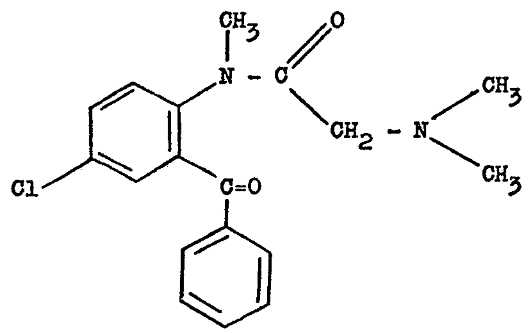

Formule brute: $C_{18}H_{19}Cl\ N_2O_2$
Masse moléculaire: 330,8
Cristaux blancs
Point de fusion: 95,5°C
Chromatographie sur plaque:
—support: gel de silice 60 F 254 Merck
—solvant: butanol—acide acétique—eau 6/2/2
—révélation: UV et iode
—Rf: 0,34
Spectre Infra-rouge: $\nu_{C-H}$ aromatique à 3060 $cm^{-1}$;
$\nu_{C=O}$ à 1660 $cm^{-1}$; $\nu_{C=C}$ à 1590 $cm^{-1}$.
Solubilités: Insoluble dans l'eau. Soluble à 20% dans l'éthanol à 95°GL et dans le diméthyl formamide.

Exemple 5
*Chlorhydrate de N-méthyl N'(méthoxy-2' éthyl) (benzoyl-2 chloro-4) glycynanilide*

D'une manière analogue à celle décrite dans l'exemple 1, mais en utilisant la méthoxy-2 éthylamine, on obtient le produit de formule:

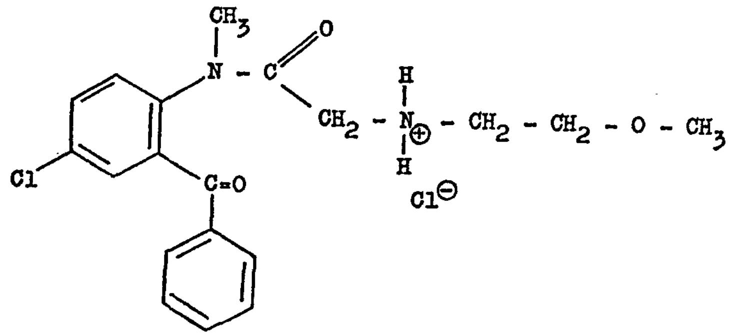

Formule brute: $C_{19}H_{22}Cl_2N_2O_3$
Masse moléculaire: 397,29
Cristaux blancs
Point de fusion: 160°C
Chromatographie sur plaque:
—support: gel de silice 60 F 254 Merck
—solvant: butanol—acid acétique—eau 6/2/2
—révélation: UV et iode
—Rf: 0,52
Spectre Infra-rouge: $\nu_{C=O}$ à 1665 $cm^{-1}$ et $\nu_{C=C}$ à 1595 $cm^{-1}$
Solubilité: soluble dans l'eau à 50%

Exemple 6

*Chlorhydrate de N-méthyl N' benzyl (benzoyl-2 chloro-4) glycynanilide*

D'une manière analogue à celle décrite dans l'exemple 1, mais en utilisant la benzylamine, on obtient le produit de formule:

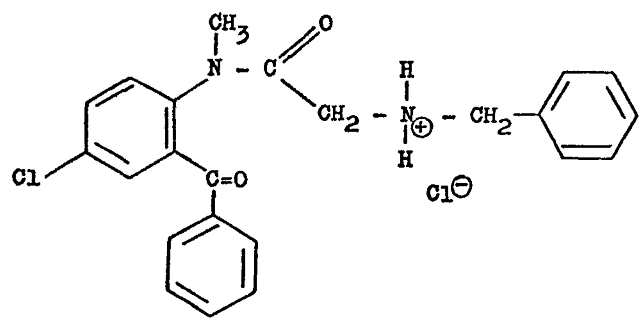

Formule brute: $C_{23}H_{22}Cl_2N_2O_2$
Masse moléculaire: 429,35
Cristaux beiges
Point de fusion: 150°C
Chromatographie sur plaque:
—support: gel de silice 60 F 254 Merck
—solvant: acétate d'éthyle
—révélation: UV et iode
—Rf: 0,23
Spectre Infra-rouge: $\nu_{C=O}$ à 1662 et 1676 $cm^{-1}$
Solubilité: soluble dans l'eau à 2,5%.

Exemple 7

*Chlorhydrate de N-méthyl N' allyl (benzoyl-2 chloro-4) glycynanilide*

D'une manière analogue à celle décrite dans l'exemple 1, mais en utilisant l'allylamine, on obtient le produit de formule:

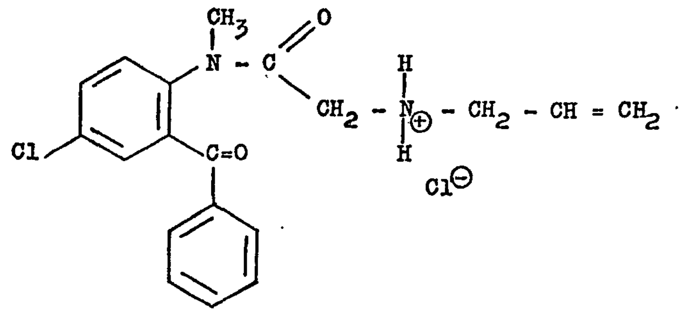

Formule brute: $C_{19}H_{20}Cl_2N_2O_2$
Masse moléculaire: 379,29
Cristaux blancs
Point de fusion: 190°C
Chromatographie sur plaque:
—support: gel de silice 60 F 254 Merck
—solvant: butanol—acide acétique—eau 6/2/2
—révélation: UV et iode
—Rf: 0,56
Spectre infra-rouge: $\nu_{C=O}$ à 1663 $cm^{-1}$ et $\nu_{C=C}$ à 1598 $cm^{-1}$
Solubilité: soluble dans l'eau à 3%.

Exemple 8
*Chlorhydrate de N-méthyl N' (diéthyl-1'-1' propargyl) (benzoyl-2 chloro-4) glycynanilide*

D'une manière analogue à celle décrite dans l'exemple 1, mais en utilisant la diéthyl-1-1 propargylamine, on obtient le produit de formule:

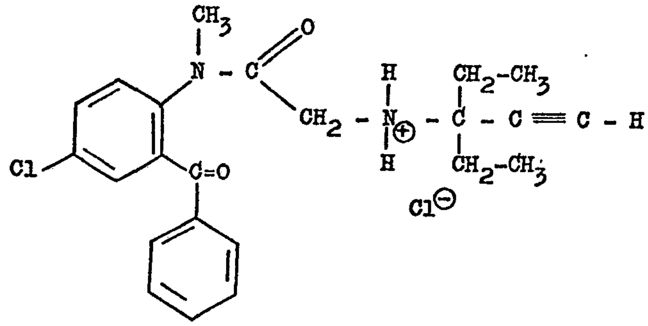

Formule brute: $C_{23}H_{26}Cl_2N_2O_2$
Masse moléculaire: 433,35
Cristaux blancs
Point du fusion: 191°C
Chromatographie sur plaque:
—support: gel de silice 60 F 254 Merck
—solvant: acétate d'éthyle
—révélation: UV et iode
—Rf: 0,73
Spectre infra-rouge: $\nu_{\equiv C-H}$ à 3165 $cm^{-1}$; $\nu_{C\equiv C}$ à 2105 $cm^{-1}$; $\nu_{C=O}$ (amide à 1690 $cm^{-1}$ et $\nu_{C=O}$ (cétone) à 1675 $cm^{-1}$.
Solubilités: Insoluble dans l'eau. Soluble à 1% dans l'éthanol à 95° GL et dans le diméthyl formamide.

Exemple 9
*Chlorhydrate de N-méthyl N'(éthynyl-1' cyclohexyl) (benzoyl-2 chloro-4) glycynanilide*

D'une manière analogue à celle décrite dans l'exemple 1, mais en utilisant l'éthynyl-1 cyclohexylamine, on obtient le produit de formule

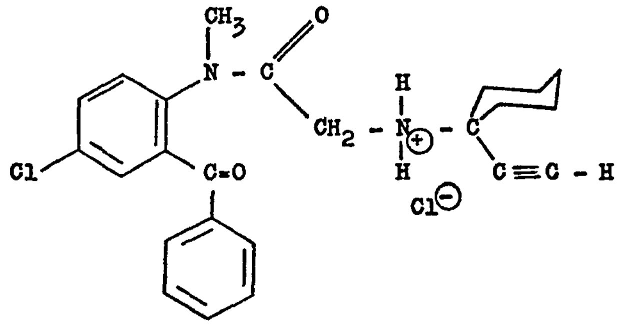

Formule brute: $C_{24}H_{25}Cl_2N_2O_2$
Masse moléculaire: 445,38
Cristaux blancs
Point de fusion: 193°C
Chromatographie sur plaque:
—support: gel de silice 60 F 254 Merck
—solvant: acétate d'éthyle—éther de pétrole 30/70
—révélation: UV et iode
Rf: 0,31
Spectre infra-rouge: $\nu_{C-H}$ à 3180 $cm^{-1}$; $\nu_{C\equiv C}$ à 2110 $cm^{-1}$; $\nu_{C=O}$ à 1675 $cm^{-1}$.
Solubilités: Insoluble dans l'eau. Soluble à 3% dans l'éthanol à 95° GL et à 2% dans le diméthyl formamide.

Exemple 10
*Chlorhydrate de N-méthyl N' cyclopropyl (benzoyl-2 chloro-4) glycynanilide*

D'une manière analogue à celle décrite dans l'exemple 1, mais en utilisant la cyclopropylamine, on obtient le produit de formule:

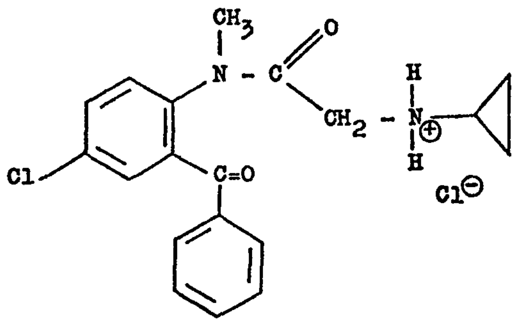

Formule brute: $C_{19}H_{20}Cl_2N_2O_2$
Masse moléculaire: 379,26
Cristaux blancs
Point de fusion: 209°C
Chromatographie sur plaque:
—support: gel de silice 60 F 254 Merck
—solvant: acétate d'éthyle
—révélation: UV et iode
—Rf: 0,27
Spectre infra-rouge: $\nu_{C=O}$ amide à 1670 $cm^{-1}$; $\nu_{C=O}$ cétone à 1655 $cm^{-1}$
Solubilité: soluble dans l'eau à 1,3%.

Exemple 11
*Chlorhydrate de N-méthyl N' cyclohexyl (benzoyl-2 chloro-4) glycynanilide*

D'une manière analogue à celle décrite dans l'exemple 1, mais en utilisant la cyclohexylamine, on obtient le produit de formule:

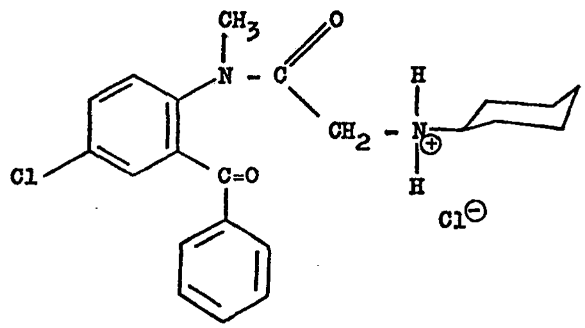

Formule brute: $C_{22}H_{26}Cl_2N_2O_2$
Masse moléculaire: 421,34
Cristaux blancs
Point de fusion: 224°C
Chromatographie sur plaque:
—support: gel de silice 60 F 254 Merck
—solvant: butanol—acide acétique—eau 6/2/2
—révélation: UV et iode
—Rf: 0,61
Spectre infra-rouge: $\nu_{C=O}$ à 1660 $cm^{-1}$ et $\nu_{C=C}$ à 1588 $cm^{-1}$
Solubilités: Insoluble dans l'eau. Soluble à 3% dans l'éthanol à 95° GL et à 1% dans le diméthyl formamide.

Exemple 12
*N-méthyl N'(diméthyl-1'-1' hydroxy-2' éthyl) (benzoyl-2 chloro-4) glycynanilide*

D'une manière analogue à celle décrite dans l'exemple 1, mais en utilisant 1'amino-2 méthyl-2 propanol, on obtient le produit de formule:

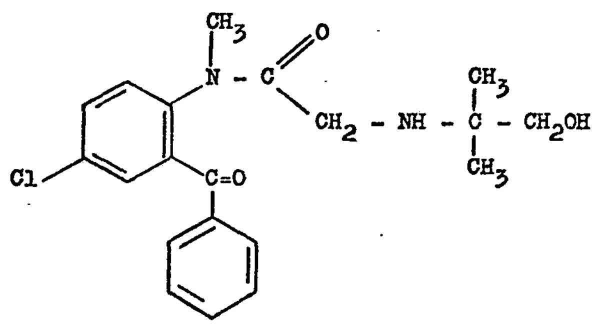

Formule brute: $C_{20}H_{23}Cl\,N_2O_3$
Masse moléculaire: 374,86
Cristaux jaune-citron
Point de fusion: 95°C
Chromatographie sur plaque:
—support: gel de silice 60 F 254 Merck
—solvant: acétate d'éthyle
—révélation: UV et iode
—Rf: 0,19
Spectre infra-rouge: $\nu_{-OH}$ à 3440 $cm^{-1}$; $\nu_{C=O}$ à 1655 $cm^{-1}$ et $\nu_{C=C}$ à 1598 $cm^{-1}$.
Solubilités: Insoluble dans l'eau. Soluble à 10% dans l'éthanol à 95° GL et à 25% dans le diméthylformamide.

Exemple 13
*Chlorhydrate de N-methyl N'(éthyl-1' hydroxyméthyl-1' hydroxy-2' éthyl) (benzoyl-2 chloro-4) glycynanilide*

D'une manière analogue à celle décrite dans l'exemple 1, mais en utilisant l'amino-2 éthyl-2 propane diol-1,3, on obtient le produit de formule:

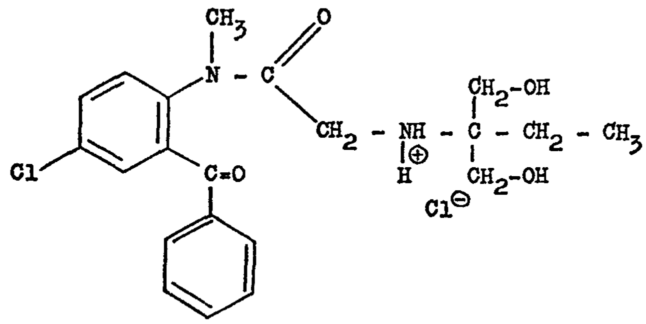

Formule brute: $C_{21}H_{26}Cl_2N_2O_4$
Masse moléculaire: 441,36
Cristaux jaune-clair
Point de fusion: 191 °C
Chromatographie sur plaque:
—support: gel de silice 60 F 254 Merck
—solvant: butanol—acide acétique—eau 6/2/2
—révélation: UV et iode
—Rf: 0,47
Spectre infra-rouge: $\nu_{-OH}$ à 3320 $cm^{-1}$; $\nu_{C=O}$ à 1660 $cm^{-1}$ et $\nu_{C=C}$ à 1596 $cm^{-1}$.
Solubilité: soluble dans l'eau à 50%.

Exemple 14
*N-N' diméthyl-N' (hydroxy-2' éthyl) (benzoyl-2 chloro-4) glycynanilide*

D'une manière analogue à celle décrite dans l'exemple 1, mais en utilisant la N-méthyl éthanolamine, on obtient le produit de formule:

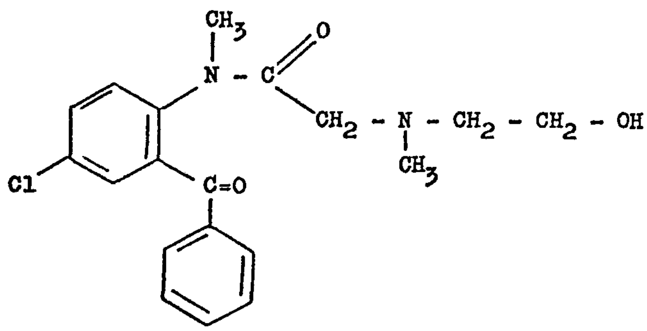

Formule brute: $C_{19}H_{21}Cl\ N_2O_3$
Masse moléculaire: 360,83
Cristaux blancs
Point de fusion: 109°C
Chromatographie sur plaque:
—support: gel de silice 60 F 254 Merck
—solvant: butanol—acide acétique—eau 6/2/2
—révélation: UV et iode
—Rf: 0,47
Spectre infra-rouge: $\nu_{-OH}$ à 3190 $cm^{-1}$; $\nu_{C=O}$ à1668 $cm^{-1}$ et $\nu_{C=C}$ à 1592 $cm^{-1}$.
Solubilités: Insoluble dans l'eau. Soluble à 20% dans l'ethanol à 95° GL et dans le diméthylformamide.

Exemple 15
*N-méthyl N' éthyl N' (hydroxy-2' éthyl) (benzoyl-2 chloro-4) glycynanilide*

D'une manière analogue à celle décrite dans l'exemple 1, mais en utilisant l'éthyl amino-2 éthanol, on obtient le produit de formule:

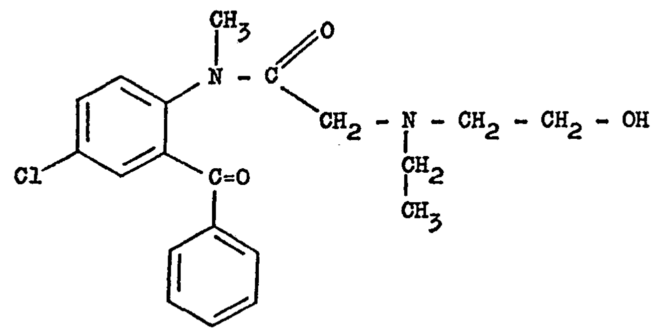

Formule brute: $C_{20}H_{23}Cl\,N_2O_3$
Masse moléculaire: 374,86
Cristaux blancs
Point de fusion: 79°C
Chromatographie sur plaque:
—support: gel de silice 60 F 254 Merck
—solvant: butanol—acide acétique—eau 6/2/2
—révélation UV et iode
—Rf: 0,35
Spectre infra-rouge: $\nu_{C=O}$ à 1668 $cm^{-1}$; $\nu_{C=C}$ à 1592 $cm^{-1}$.
Solubilités: Insoluble dans l'eau. Soluble à 10% dans l'éthanol à 95° GL et à 30% dans le diméthyl formamide.

Exemple 16
*Chlorhydrate de N-méthyl N'-N' bis (hydroxy-2' éthyl) (benzoyl-2 chloro-4) glycynanilide*

D'une manière analogue à celle décrite dans l'exemple 1, mais en utilisant la diéthanolamine, on obtient le produit de formule:

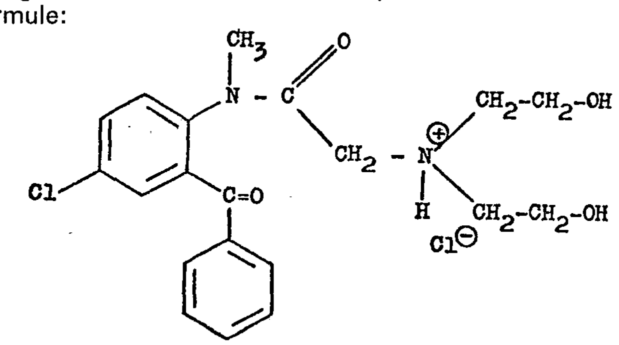

Formule brute: $C_{20}H_{24}Cl_2N_2O_4$
Masse moléculaire: 427,33
Cristaux blancs
Point de fusion: 174°C
Chromatographie sur plaque:
—support: gel de silice 60 F 254 Merck
—solvant: butanol—acide acétique—eau 6/2/2
—rélévation: UV et iode
—Rf: 0,35
Spectre infra-rouge: $\nu_{C=O}$ à 1669 $cm^{-1}$ et $\nu_{C=C}$ à 1596 $cm^{-1}$.
Solubilité: soluble dans l'eau à 5%.

Exemple 17
*Chlorhydrate de N-méthyl pipéridino-2 (benzoyl-2' chloro-4') acétanilide*

Comme dans l'exemple 1, mais en utilisant la pipéridine, on obtient le produit de formule

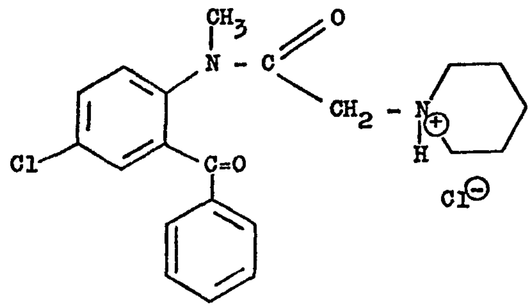

Formule brute: $C_{21}H_{24}Cl_2N_2O_2$
Masse moléculaire: 407,34
Cristaux blancs
Point de fusion: 140°C
Chromatographie sur plaque:
—support: gel de silice 60 F 254 Merck
—solvant: butanol—acide acétique—eau 6/2/2
—révélation: UV et iode
—Rf: 0,55
Spectre infra-rouge: $\nu_{C=O}$ (amide) à 1680 $cm^{-1}$; $\nu_{C=O}$ (cétone) à 1667 $cm^{-1}$; et $\nu_{C=C}$ à 1592 $cm^{-1}$.
Solubilité: soluble dans l'eau à 50%.

Exemple 18

*Chlorhydrate de N-méthyl morpholino-2 (benzoyl-2' chloro-4') acétanilide*

D'une manière analogue à celle décrite dans l'exemple 1, mais en utilisant la morpholine, on obtient le produit de formule:

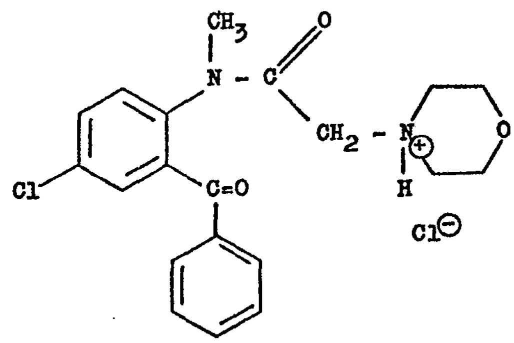

Formule brute: $C_{20}H_{22}Cl_2N_2O_3$
Masse moléculaire: 409,3
Cristaux blancs
Point de fusion: 172°C
Chromatographie sur plaque:
—support: gel de silice 60 F 254 Merck
—solvant: butanal—acide acétique—eau 6/2/2
—révélation: UV et iode
—Rf: 0,61
Spectre infra-rouge: $\nu_{C=O}$ à 1663 $cm^{-1}$ et $\nu_{C=C}$ à 1595 $cm^{-1}$
Solubilité: soluble dans l'eau à 10%.

Exemple 19

*Chlorhydrate de N-méthyl N' (méthyl-2' allyl) (benzoyl-2 chloro-4) glycynanilide*

D'une manière analogue à celle décrite dans l'exemple 1, mais en utilisant la méthyl-2 allylamine, on obtient le produit de formule:

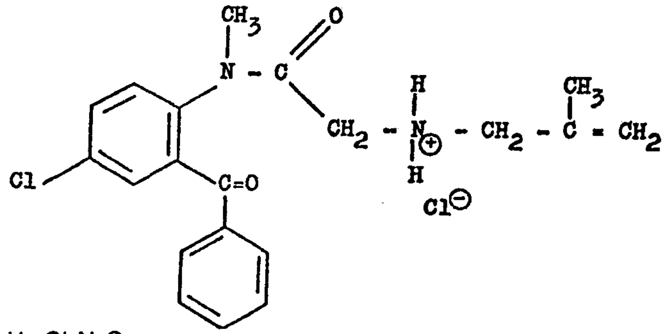

Formule brute: $C_{20}H_{22}Cl_2N_2O_2$
Masse moléculaire: 393,32
Cristaux blancs
Point de fusion: 166°C
Chromatographie sur plaque:
—support: gel de silice 60 F 254 Merck
—solvant: butanol—acide acétique—eau 6/2/2
—révélation: UV et iode
—Rf: 0,54
Solubilité: soluble dans l'eau à 5%.

Exemple 20

*Chlorhydrate de N-N' diméthyl N' cyclohexyl (benzoyl-2 chloro-4) glycynanilide*

D'une manière analogue à celle décrite dans l'exemple 1, mais en utilisant la N-méthyl cyclohexylamine, on obtient le produit de formule:

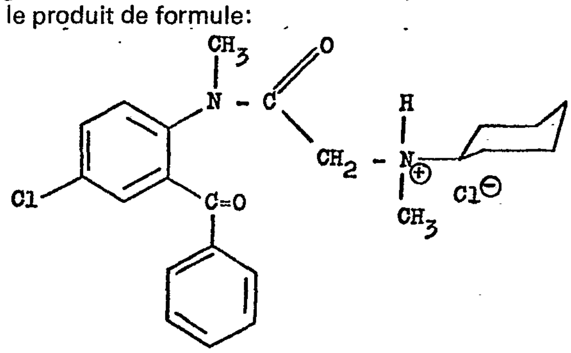

Formule brute: $C_{23}H_{28}Cl_2N_2O_2$
Masse moléculaire: 435,4
Cristaux blancs
Point de fusion: 141°C
Chromatographie sur plaque:
—support: gel de silice 60 F 254 Merck
—solvant: butanol—acide acétique—eau 6/2/2
—révélation: UV et iode
Rf: 0,43
Solubilité: Soluble dans l'eau à 2%.

Exemple 21

*Chlorhydrate de N-méthyl N' isopropyl N' cyclohexyl (benzoyl-2 chloro-4) glycynanilide*

D'une manière analogue à celle décrite dans l'exemple 1, mais en utilisant la N-isopropyl cyclohexylamine, on obtient le produit de formule:

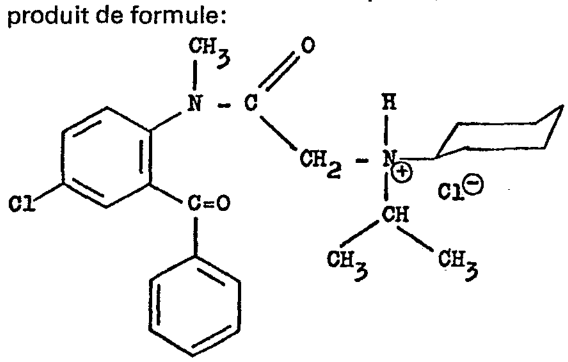

Formule brute: $C_{25}H_{32}Cl_2N_2O_2$
Masse moléculaire: 463,45
Cristaux jaunes
Point de fusion: 145°C
Chromatographie sur plaque:
—support: gel de silice 60 F 254 Merck
—solvant: butanol—acide acétique—eau 6/2/2
—révélation: UV et iode
Rf: 0,51
Solubilité: soluble dans l'eau à 5%.

Exemple 22
*N-méthyl méthyl-4' pipérazino-2 (benzoyl-2 chloro-4) acétanilide*

D'une maniére analogue à celle décrite dans l'exemple 1, mais en utilisant la N-méthyl pipérazine, on obtient le produit de formule:

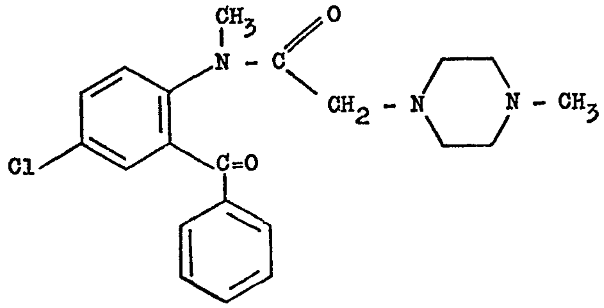

Formule brute: $C_{21}H_{24}Cl\ N_3O_2$
Masse moléculaire: 385,89
Cristaux blancs
Point de fusion: 146°C
Chromatographie sur plaque:
—support: gel de silice 60 F 254 Merck
—solvant: butanol—acide acétique—eau 6/2/2
—révélation: UV et iode
—Rf: 0,20
Solubilités: Insoluble dans l'eau. Soluble à 5% dans l'éthanol à 95° GL et dans le diméthyl formamide.

Exemple 23
*N-méthyl imidazolyl-2 (benzoyl-2 chloro-4) acétanilide*

D'une manière analogue à celle décrite dans l'exemple 1, mais en utilisant l'imidazol, on obtient le produit de formule:

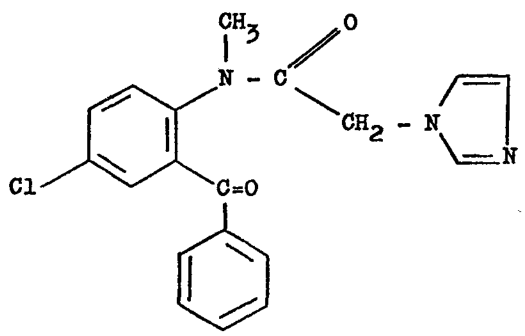

Formule brute: $C_{19}H_{16}Cl\ N_3O_2$
Masse moléculaire: 353,81
Cristaux blancs
Point de fusion: 143°C
Chromatographie sur plaque:
—support: gel de silice 60 F 254 Merck
—solvent: butanol-acide acétique—eau 6/2/2
—révélation: UV et iode
—Rf: 0,39
Solubilités: Insoluble dans l'eau. Soluble à 10% dans l'éthanol à 95° GL et à 20% dans le diméthylformamide.

Les composés de la présente invention, doués d'une remarquable activité sur le système nerveux central, sont donc susceptibles d'être administrés à l'homme ou à l'animal: par voie orale ou par injection, sous la forme d'une base libre ou bien de l'un de ses sels thérapeutiquement acceptables.

A titre de simple illustration on indiquera ci-après quelques résultats des divers essais toxicologiques et pharmacologiques effectués sur les composés de l'invention.

a) *Etude de toxicité*

Les composés de la présente invention ont été soumis à des contrôles de toxicité. La toxicité de certains composés déterminée par la dose létale 50 est rapportée dans le tableau suivant. Elle a été recherchée sur des lots de 10 souris par voie orale, intra-péritonéale et intra-veineuse dans certains cas, et calculée selon la méthode de MILLER et TAINTER (Proc. Soc. expér. Biol. Méd., 1944, 57,261).

Résultats

| Composé de l'exemple n°. | $DL_{50}$ voie orale en mg/kg | $DL_{50}$ voie I.P. en mg/kg | $DL_{50}$ voie I.V. en mg/kg |
|---|---|---|---|
| 2 | > 1 000 | 450 | – |
| 5 | 750 | 300 | – |
| 7 | > 1 000 | 500 | – |
| 8 | 1 000 | 400 | – |
| 9 | 560 | 200 | – |
| 11 | 1 050 | 305 | 50 |
| 14 | 1 200 | 350 | 55 |
| 15 | 550 | 150 | – |
| 16 | > 1 000 | > 500 | – |
| 18 | 1 700 | 750 | 103 |
| 19 | 1 500 | 500 | 60 |
| 20 | > 1 000 | > 500 | – |
| 21 | 1 000 | 500 | – |
| 22 | 1 000 | 500 | – |
| 23 | 1 000 | 500 | – |

b) *Propriétés pharmacologiques*

*Activité antagoniste au pentaméthylènetétrazole*

On réalise cet essai sur un groupe de 10 souris mâles de souche Swiss. Dans un délai de 15 minutes après l'injection sous-cutanée de 125 mg/kg de pentaméthylènetétrazole, les souris ont des convulsions toniques dont l'issue est fatale.

Pour l'essai, on administre le composé par voie orale 60 minutes avant l'injection de pentaméthylènetétrazole. Les animaux sont observés pendant 2 heures après administration du pentaméthylènetétrazole. Dans certains cas particuliers les assais ont été confirmés par voie intra-péritonéale. Les résultats sont exprimés par la dose efficace $DE_{50}$ (Goodman et Col.—J. Pharmacol. *108*, 1953).

Résultats

| Composé de l'exemple n° | Activité antagoniste au pentaméthylènetétrazole $DE_{50}$ – mg/kg P.O. | |
|---|---|---|
| | P.O. | I.P. |
| 2 | 1,5 | – |
| 5 | 2,6 | – |
| 7 | 1,9 | – |
| 8 | 4,1 | – |
| 9 | 3,9 | – |
| 10 | 0,8 | – |
| 11 | 1,35 | 1,25 |
| 14 | 1,7 | 1,5 |
| 15 | 2,9 | – |
| 16 | 3,4 | – |
| 18 | 3,3 | 1,4 |
| 19 | 1,7 | 1,1 |
| 20 | 1,9 | – |
| 21 | 2,2 | – |
| Diazépam | 1 | – |
| Chlordiazépoxyde | 5 | – |

*Activité dans l'essai sur tige tournante* (Rota Rod)

On réalise cet essai sur des souris mâles de souche Swiss. On place la souris sur une tige de bois d'un diamètre de 3 cm, tournant à raison de 5 tours/minute. On choisit les souris qui peuvent rester sur la tige pendant au moins 3 minutes au cours d'essais successifs et on les rassamble par groupe de 10 pour l'essai de chaque dose. Si la souris tombe de la tige en moins de 2 minutes, on considère que le composé essayé est efficace. On exprime les résultats par la dose efficace $DE_{50}$ selon N. W. DUNHAN et T.S. MIVA—J. amer. pharm. Ass., 1957, 46, 208.

Résultats

| Composé de l'exemple n° | Rota Rod $DE_{50}$ V.O. |
|---|---|
| 11 | 27 |
| 14 | 66 |
| 18 | 60 |
| 19 | 21 |

Compte tenu de leurs propriétés pharmacologiques, les composés de l'invention, et plus particulièrement les composés des exemples 2, 10, 11, 14, 19 et 20 peuvent être utilisés en thérapeutique dans le traitement de l'anxiété et des névroses.

Ces composés et leurs sels d'addition d'acides thérapeutiquement compatibles peuvent être utilisés comme médicaments, par exemple sous forme de préparations pharmaceutiques adaptées à l'administration entérale ou parentérale, avec par exemple l'eau, le lactose, la gélatine, les amidons, le stéarate de magnésium, le talc, les huiles végétales, les gommes, les polyalcoylèneglycols, la vaseline, etc.

Ces préparations peuvent se présenter sous forme solide, par exemple de comprimés, dragées, capsules, etc. ou sous forme liquide, par exemple de solutions, suspensions ou émulsions.

Les préparations pharmaceutiques sous une forme appropriée à l'injection sont préférées. Ces préparations peuvent être soumises à des opérations pharmaceutiques classiques telles que stérilisation et/ou peuvent contenir des adjuvants par exemple des agents conservateurs, stabilisants de mouillage ou d'émulsification, des composés-tampons, etc.

Les dosages, auxquels les composés actifs et leurs sels d'addition d'acide thérapeutiquement compatibles peuvent être administrés, peuvent varier dans des proportions importantes selon l'état du patient. Un dosage quotidien d'environ 0,01 mg à 1 mg par kg de poids corporel est toutefois préféré.

Les compositions pharmaceutiques selon l'invention peuvent être utilisées en médecine interne, par exemple dans le traitement d'états pathologiques organiques, tels que l'hypertension artérielle et les coronarites, accompagnés et aggravés par un état anxieux; en médecine psychosomatique, par exemple pour le traitement de l'asthme, des ulcères gastrodudodénaux, des colopathies et d'autres affections digestives fonctionnelles; ainsi qu'en psychiatrie, par exemple pour le traitement d'états d'agitation anxieux chez les sujets psychotiques.

Bien entendu, la présente invention ne se trouve pas limitée aux exemples particuliers mentionnés à simple titre illustratif, mais il est parfaitement possible, sans pour autant sortir du cadre de l'invention, d'en imaginer un certain nombre de variantes et de modifications.

**Revendications**

1. Nouveaux dérivés de benzoyl-2 chloro-4 glycynanilides répondant à la formule générale I

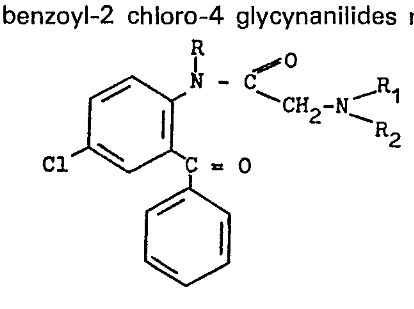

(I)

dans laquelle

R représente un groupe alcoyle linéaire ou ramifié, de préférence un groupe alcoyle inférieur;

$R_1$ et $R_2$ peuvent être identiques ou différents et sont choisis parmi l'hydrogène, les groupes alcoyle, alcényle, alcynyle, hydroxyalcoyle, alcoxyalcoyle, aralcoyle, ces divers groupes pouvant être linéaires ou ramifiés, et parmi les groupes cycloalcoyle ayant de 3 à 6 chaînons et étant éventuellement substitués sur le carbone en $\alpha$ par un radical alcynyle;

les groupes $R_1$ et $R_2$ pouvant en outre former avec l'atome d'azote auquel ils sont reliés un hétérocycle

azoté à 5 ou 6 chaînons, saturé ou non, et comprenant éventuellement un second hétéroatome choisi parmi l'oxygène et l'azote, ledit hétérocycle étant éventuellement substitué, de préférence sur le second atome d'azote, par un radical alcoyle inférieur;
ainsi que leurs sels obtenus avec des acides minéraux ou organiques thérapeutiquement acceptables.

2. Composés de formule générale I selon la revendication 1, caractérisés par le fait que R représente le radical méthyle.

3. Composé de formule générale I selon la revendication 2, caractérisé en ce qu'il s'agit du chlorhydrate de N-méthyl N' diméthyl-1-1 propargyl (benzoyl-2 chloro-4) glycynanilide.

4. Composé de formule générale I selon la revendication 2, caractérisé en ce qu'il s'agit du chlorhydrate de N-méthyl N' (méthoxy-2' éthyl) (benzoyl-2 chloro-4) glycynanilide.

5. Composé de formule générale I selon la revendication 2, caractérisé en ce qu'il s'agit du chlorhydrate de N-méthyl N'-allyl (benzoyl-2 chloro-4) glycynanilide.

6. Composé de formule générale I selon la revendication 2, caractérisé en ce qu'il s'agit du chlorhydrate de N-méthyl N' (diéthyl-1'-1' propargyl) (benzoyl-2 chloro-4) gylcynanilide.

7. Composé de formule générale I selon la revendication 2, caractérisé en ce qu'il s'agit du chlorhydrate de N-méthyl N' (éthynyl-1' cyclohexyl) (benzoyl-2 chloro-4) glycynanilide.

8. Composé de formule générale I selon la revendication 2, caractérisé en ce qu'il s'agit du chlorhydrate de N-methyl N' cyclopropyl (benzoyl-2 chloro-4) glycynanilide.

9. Composé de formule générale I selon la revendication 2, caractérisé en ce qu'il s'agit du chlorhydrate de N-méthyl N' cyclohexyl (benzoyl-2 chloro-4) glycynanilide.

10. Composé de formule générale I selon la revendication 2, caractérisé en ce qu'il s'agit du N—N' diméthyl N' (hydroxy-2' éthyl) (benzoyl-2 chloro-4) glycynanilide.

11. Composé de formule générale I selon la revendication 2, caractérisé en ce qu'il s'agit du N-méthyl N'éthyl N' (hydroxy-2' éthyl) (benzoyl-2 chloro-4) glycynanilide.

12. Composé de formule générale I selon la revendication 2, caractérisé en ce qu'il s'agit du chlorhydrate de N-méthyl N' bis (hydroxy-2' éthyl) (benzyl-2 chloro-4) glycynanilide.

13. Composé de formule générale I selon la revendication 2, caractérisé en ce qu'il s'agit du chlorhydrate de N-méthyl pipéridino-2 (benzoyl-2' chloro-4) acétanilide.

14. Composé de formule générale I selon la revendication 2, caractérisé en ce qu'il du chlorhydrate de N-méthyl morpholino-2 (benzoyl-2' chloro-4') acétanilide.

15. Composé de formule générale I selon la revendication 2, caractérisé en ce qu'il s'agit du chlorhydrate de N-méthyl N' (méthyl-2' allyl) (benzoyl-2 chloro-4) glycynanilide.

16. Composé de formule générale I selon la revendication 2, caractérisé en ce qu'il s'agit du chlorhydrate de N-N' diméthyl N' cyclohexyl (benzoyl-2 chloro-4) glycynanilide.

17. Composé de formule générale I selon la revendication 2, caractérisé en ce qu'il s'agit du chlorhydrate de N-méthyl N' isopropyl N' cyclohexyl (benzoyl-2 chloro-4) glycynanilide.

18. Composé de formule générale I selon la revendication 2, caractérisé en ce qu'il s'agit du N-méthyl méthyl-4' pipérazino-2 (benzoyl-2' chloro-4') acétanilide.

19. Composé de formule générale I selon la revendication 2, caractérisé en ce qu'il s'agit du N-méthyl imidazolyl-2 (benzoyl-2' chloro-4') acétanilide.

20. Procédé de préparation des composés de formule générale I selon l'une des revendications 1 à 19, caractérisé par le fait qu'il consiste à faire réagir un halo-acétamide de formule générale II

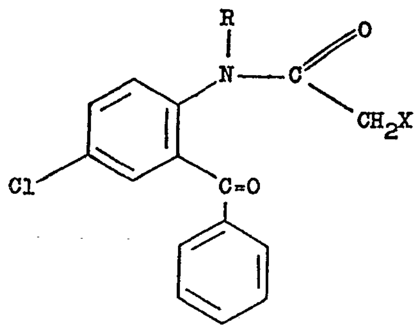

(II)

dans laquelle:
R a la signification donnée à la revendication 1 et
X représente un atome d'halogène,
avec une amine de formula III

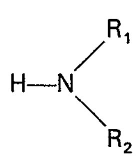

(III)

dans laquelle:

$R_1$ et $R_2$ ont les significations données à la revendication 1.

21. Procédé selon la revendication 20, caractérisé par le fait que l'halo-acétamide de formule II est obtenu par réaction d'un halogénure d'halo-acétyle de formule IV

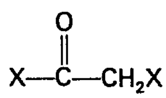

(IV)

sur un dérivé N-substitué de l'amino-2 chloro-5 benzophénone de formule V

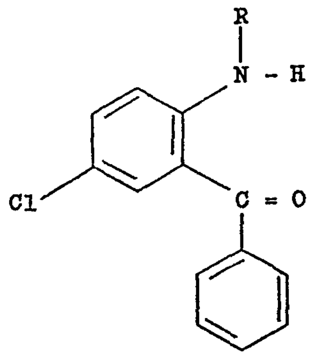

(V)

dans laquelle:

R et X ont les significations indiquées à la revendication 20.

22. Procédé selon la revendication 21, caractérisé par le fait que le dérivé N-substitué de l'amino-2 chloro-5 benzophénone de formule V est obtenu par hydrolyse d'un composé N-substitué de tosylamido-2 chloro-5 benzophénone de formule VI

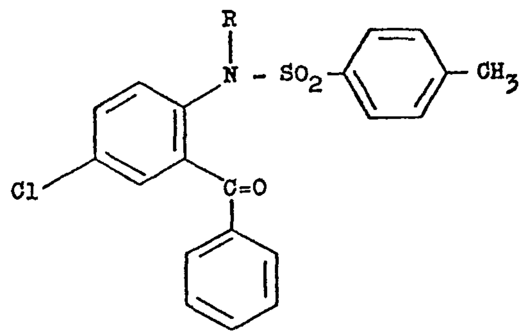

(VI)

R ayant la signification donnée à la revendication 20.

23. Procédé selon la revendication 22, caractérisé par le fait que le composé N-substitué de tosylamido-2 chloro-5 benzophénone de formule VI est obtenu par réaction d'un halogénure de formule VII

$$R—X \qquad (VII)$$

sur la tosylamido-2 chloro-5 benzophénone,

R et X ayant les significations données à la revendication 20.

24. Procédé selon la revendication 23, caractérisé par le fait que la tosylamido-2 chloro-5 benzophénone est obtenue par réaction d'un halogénure de tosyle sur l'amino-2 chloro-5 benzophénone.

25. Procédé selon la revendication 21, caractérisé par le fait que le dérivé N-substitué de l'amino-2 chloro-5 benzophénone de formule V est obtenu par réaction d'un sulfate de formule VIII

$$(R)_2SO_4 \qquad (VIII)$$

dans laquelle R a la signification donnée à la revendication 20,

sur l'amino-2 chloro-5 benzophénone, en présence d'acide acétique.

26. A titre de médicaments nouveaux, les composés de formule générale I selon l'une des revendications 1 à 19, ainsi que leurs sels d'addition avec des acides physiologiquement acceptables.

27. Compositions pharmaceutiques, caractérisées par le fait qu'elles contiennent, à titre de substance active, au moins un composé de formule générale I selon l'une des revendications 1 à 19, ou l'un de leurs sels d'addition avec des acides physiologiquement acceptables.

**Claims**

1. New 2-benzoyl-4-chloroglycyanilide derivatives corresponding to general formula I below

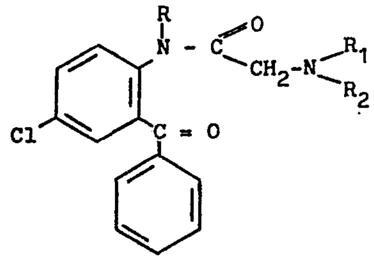

(I)

in which

R represents a linear or branched alkyl group, preferably a lower alkyl group;

$R_1$ and $R_2$ may be the same or different and are selected from hydrogen, alkyl, alkenyl, alkynyl, hydroxy alkyl, alkoxy alkyl and aralkyl groups, these various groups optionally being linear or branched, and from 3- to 6-membered cycloalkyl groups optionally substituted on the $\alpha$-carbon atom by an alkynyl radical;

in addition, the groups $R_1$ and $R_2$ may form with the nitrogen atom to which they are attached an optionally saturated 5-membered or 6-membered nitrogen heterocycle optionally containing a second heteroatom selected from oxygen and nitrogen, said heterocycle optionally being substituted, preferably on the second nitrogen atom, by a lower alkyl radical; and their salts obtained with therapeutically acceptable mineral or organic acids.

2. Compounds of general formula I as claimed in Claim 1, characterised in that R represents the methyl radical.

3. A compound of general formula I as claimed in Claim 2, characterised in that it is the hydrochloride of N - methyl - N' - dimethyl - 1,1 - propargyl - (2 - benzoyl - 4 - chloro) - glycynanilide.

4. A compound of general formula I as claimed in Claim 2, characterised in that it is the hydrochloride of N - methyl - N' - (2' - methoxy - ethyl) - (2 - benzoyl - 4 - chloro) - glycynanilide.

5. A compound of general formula I as claimed in Claim 2, characterised in that it is the hydrochloride of N - methyl - N' - allyl - (2 - benzoyl - 4 - chloro) - glycynanilide.

6. A compound of general formula I as claimed in Claim 2, characterised in that it is the hydrochloride of N - methyl - N' - (1',1' - diethyl - propargyl) - (2 - benzoyl - 4 - chloro) - glycynanilide.

7. A compound of general formula I as claimed in Claim 2, characterised in that it is the hydrochloride of N - methyl - N' - (1' - ethynyl - cyclohexyl) - (2 - benzoyl - 4 - chloro) - glycynanilide.

8. A compound of general formula I as claimed in Claim 2, characterised in that it is the hydrochloride of N - methyl - N' - cyclopropyl - (2 - benzoyl - 4 - chloro) - glycynanilide.

9. A compound of general formula I as claimed in Claim 2, characterised in that it is the hydrochloride of N - methyl - N' - cyclohexyl - (2 - benzoyl - 4 - chloro) - glycynanilide.

10. A compound of general formula I as claimed in Claim 2, characterised in that it is N,N' - dimethyl - N' - (2' - hydroxyethyl) - (2 - benzoyl - 4 - chloro) - glycynanilide.

11. A compound of general formula I as claimed in Claim 2, characterised in that it is N - methyl - N' - ethyl - N' - (2' - hydroxyethyl) - (2 - benzoyl - 4 - chloro) - glycynanilide.

12. A compound of general formula I as claimed in Claim 2, characterised in that it is the hydrochloride of N - methyl - N' - bis - (2' - hydroxyethyl) - (2 - benzoyl - 4 - chloro) - glycynanilide.

13. A compound of general formula I as claimed in Claim 2, characterised in that it is the hydrochloride of N - methyl - 2 - piperidino - (2' - benzoyl - 4' - chloro) - acetanilide.

14. A compound of general formula I as claimed in Claim 2, characterised in that it is the hydrochloride of N - methyl - 2 - morpholino - (2' - benzoyl- 4' - chloro) - acetanilide.

15. A compound of general formula I as claimed in Claim 2, characterised in that it is the hydrochloride of N - methyl - N' - (2' - methyl - allyl) - (2 - benzoyl - 4 - chloro) - glycynanilide.

16. A compound of general formula I as claimed in Claim 2, characterised in that it is the hydrochloride of N,N' - dimethyl - N' - cyclohexyl - (2 - benzoyl - 4 - chloro) - glycynanilide.

17. A compound of general formula I as claimed in Claim 2, characterised in that it is the hydrochloride of N - methyl - N' - isopropyl - N' - cyclohexyl) - (2 - benzoyl - 4 - chloro) - glycynanilide.

18. A compound of general formula I as claimed in Claim 2, characterised in that it is N - methyl - 4' - methyl - 2 - piperazino - (2' - benzoyl - 4' - chloro) - acetanilide.

19. A compound of general formula I as claimed in Claim 2, characterised in that it is N - methyl - 2 - imidazolyl - (2' - benzoyl - 4' - chloro) - acetanilide.

20. A process for preparing the compounds of general formula I claimed in any of Claims 1 to 19, characterised in that it comprises reacting a haloacetamide corresponding to general formula II below

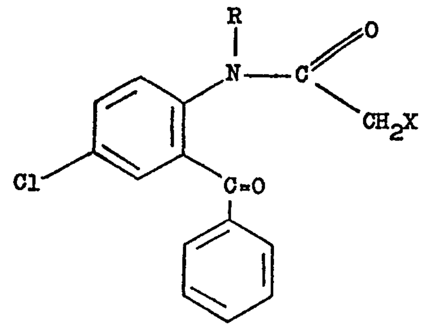

(II)

in which
R is as defined in Claim 1 and
X represents a halogen atom,
with an amine corresponding to formula III below

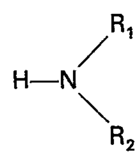

in which
$R_1$ and $R_2$ are as defined in Claim 1.

21. A process as claimed in Claim 20, characterised in that the halo-acetamide of formula II is obtained by reacting a halo-acetyl halide corresponding to formula IV below

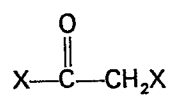

(IV)

with an N-substituted derivative of 2 - amino - 5 - chlorobenzophenone corresponding to formula V below

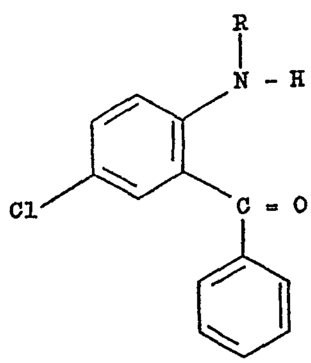

(V)

in which
R and X are as defined in Claim 20.

22. A process as claimed in Claim 21, characterised in that the N-substituted derivative of 2 - amino - 5 - chlorobenzophenone of formula V is obtained by the hydrolysis of an N-substituted compound of 2 - tosylamido - 5 - chlorobenzophenone corresponding to formula VI below

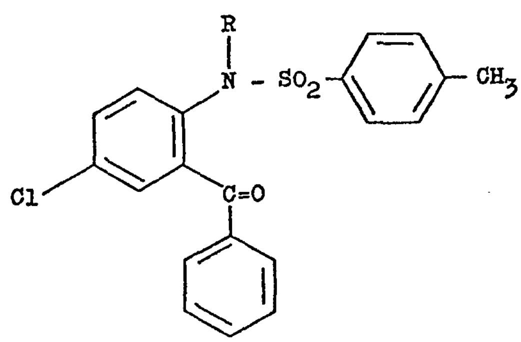

(VI)

in which R is as defined in Claim 20.

23. A process as claimed in Claim 22, characterised in that the N-substituted compound of 2 - tosylamido - 5 - chlorobenzophenone corresponding to formula VI is obtained by reacting a halide corresponding to formula VII below

$$R—X \qquad (VII)$$

in which R and X are as defined in Claim 20, with 2 - tosylamido - 5 - chlorobenzophenone.

24. A process as claimed in Claim 23, characterised in that the 2 - tosylamido - 5 - chlorobenzophenone is obtained by reacting a tosyl halide with 2 - amino - 5 - chlorobenzophenone.

25. A process as claimed in Claim 21, characterised in that the N-substituted derivative of 2 - amino - 5 - chlorobenzophenone corresponding to formula V is obtained by reacting a sulphate corresponding to formula VIII below

$$(R)_2SO_4 \qquad (VIII)$$

in which R is as defined in Claim 20, with 2 - amino - 5 - chlorobenzophenone in the presence of acetic acid.

26. As new medicaments, the compounds corresponding to general formula I claimed in any of Claims 1 to 19 and their acid addition salts with physiologically acceptable acids.

27. Pharmaceutical compositions, characterised in that they contain as active substance at least one compound of general formula I as claimed in any of Claims 1 to 19 or one of its acid addition salts with physiologically acceptable acids.

**Patentansprüche**

1. Neue Derivate von 2 - Benzoyl - 4 - chlor - glycinaniliden der allgemeinen Formel I

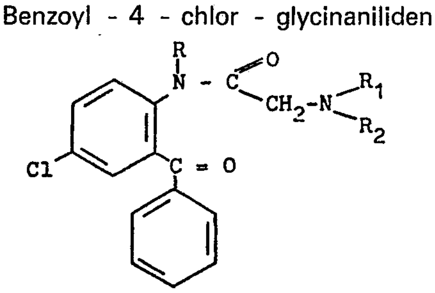

(I)

worin

R eine lineare oder verzweigte Alkylgruppe, vorzugsweise eine niedere Alkylgruppe darstellt;

$R_1$ und $R_2$ identisch oder verschieden sein können und ausgewählt werden aus Wasserstoff, Alkyl-, Alkenyl-, Alkinyl-, Hydroxyalkyl-, Alkoxyalkyl-, Aralkyl-Gruppen, wobei diese verschiedenen Gruppen linear oder verzweigt sein können, und 3- bis 6-gliedrigen Cycloalkyl-Gruppen, die an dem Kohlenstoffatom in $\alpha$-Stellung gegebenenfalls durch einen Alkinylrest substituert sein können;

wobei die Gruppen $R_1$ und $R_2$ außerdem zusammen mit dem Stickstoffatom, an das sie gebunden sind, einen gesättigten oder ungesättigten, 5-oder 6-gliedrigen, stickstoffhaltigen Heterocyclus bilden können, der gegebenenfalls ein zweites Heteroatom, ausgewählt aus Sauerstoff und Stickstoff, enthält und gegebenenfalls, vorzugsweise an dem zweiten Stickstoffatom, durch einen niederen Alkylrest substituiert ist;

sowie ihre Salze, die mit therapeutisch akzeptablen Mineralsäuren oder organischen Säuren erhalten werden.

2. Verbindungen der allgemeinen Formel I nach Anspruch 1, dadurch gekennzeichnet, daß R den Methylrest bedeutet.

3. Verbindung der allgemeinen Formel I nach Anspruch 2, dadurch gekennzeichnet, daß es sich dabei handelt um das Hydrochlorid von N - Methyl - N' - 1,1 - dimethyl - propargyl - (2 - benzoyl - 4 - chlor)glycinanilid.

4. Verbindung der allgemeinen Formel I nach Anspruch 2, dadurch gekennzeichnet, daß es sich dabei handelt um das Hydrochlorid von N - Methyl - N' - (2' - methoxy - äthyl) - (2 - benzoyl - 4 - chlor)glycinanilid.

5. Verbindung der allgemeinen Formel I nach Anspruch 2, dadurch gekennzeichnet, daß es sich dabei handelt um das Hydrochlorid von N - Methyl - N' - allyl - (2 - benzoyl - 4 - chlor)glycinanilid.

6. Verbindung der allgemeinen Formel I nach Anspruch 2, dadurch gekennzeichnet, daß es sich dabei handelt um das Hydrochlorid von N - Methyl - N' - (1',1' - diäthyl - propargyl) - (2 - benzoyl - 4 - chlor)glycinanilid.

7. Verbindung der allgemeinen Formel I nach Anspruch 2, dadurch gekennzeichnet, daß es sich

dabei handelt um das Hydrochlorid von N - Methyl - N' - (1' - äthinyl - cyclohexyl) - (2 - benzoyl - 4 - chlor)glycinanilid.

8. Verbindung der allgemeinen Formel I nach Anspruch 2, dadurch gekennzeichnet, daß es sich dabei handelt um das Hydrochlorid von N - Methyl - N' - cyclopropyl - (2 benzoyl - 4 - chlor)glycinanilid.

9. Verbindung der allgemeinen Formel I nach Anspruch 2, dadurch gekennzeichnet, daß es sich dabei handelt um das Hydrochlorid von N - Methyl - N' - cyclohexyl - (2 - benzoyl - 4 - chlor)glycinanilid.

10. Verbindung der allgemeinen Formel I nach Anspruch 2, dadurch gekennzeichnet, daß es sich dabei handelt um N,N' - Dimethyl - N' - (2' - hydroxy - äthyl) - (2 - benzoyl - 4 - chlor)glycinanilid.

11. Verbindung der allgemeinen Formel I nach Anspruch 2, dadurch gekennzeichnet, daß es sich dabei handelt um N - Methyl - N' - äthyl - N' - (2' - hydroxy - äthyl) - (2 - benzoyl - 4 - chlor)-glycinanilid.

12. Verbindung der allgemeinen Formel I nach Anspruch 2, dadurch gekennzeichnet, daß es sich dabei handelt um das Hydrochlorid von N-Methyl-N'-bis-(2'-hydroxy-äthyl)-(2-benzoyl-4-chlor)glycinanilid.

13. Verbindung der allgemeinen Formel I nach Anspruch 2, dadurch gekennzeichnet, daß es sich dabei handelt um das Hydrochlorid von N - Methyl - 2 - piperidino - (2' - benzoyl - 4' - chlor)acetanilid.

14. Verbindung der allgemeinen Formel I nach Anspruch 2, dadurch gekennzeichnet, daß es sich dabei handelt um das Hydrochlorid von N - Methyl - 2 - morpholino - (2' - benzoyl - 4' - chlor)acetanilid.

15. Verbindung der allgemeinen Formel I nach Anspruch 2, dadurch gekennzeichnet, daß es sich dabei handelt um das Hydrochlorid von N - Methyl - N' - (2' - methyl - allyl) - (2 - benzoyl - 4 - chlor)glycinanilid.

16. Verbindung der allgemeinen Formel I nach Anspruch 2, dadurch gekennzeichnet, daß es sich dabei handelt um das Hydrochlorid von N,N' - Dimethyl - N' - cyclohexyl - (2 - benzoyl - 4 - chlor)-glycinanilid.

17. Verbindung der allgemeinen Formel I nach Anspruch 2, dadurch gekennzeichnet, daß es sich dabei handelt um das Hydrochlorid von N - Methyl - N' - isopropyl - N' - cyclohexyl - (2 - benzoyl - 4 - chlor)glycinanilid.

18. Verbindung der allgemeinen Formel I nach Anspruch 2, dadurch gekennzeichnet, daß es sich dabei handelt um N - Methyl - 4' - methyl - 2 - piperazino - (2' - benzoyl - 4' - chlor)acetanilid.

19. Verbindung der allgemeinen Formel I nach Anspruch 2, dadurch gekennzeichnet, daß es sich dabei handelt um N - Methyl - 2 - imidazolyl - (2' - benzoyl - 4' - chlor)acetanilid.

20. Verfahren zur Herstellung der Verbindungen der allgemeinen Formel I nach einem der Ansprüche 1 bis 19, dadurch gekennzeichnet, daß man.

ein Halogenacetamid der allgemeinen Formel II

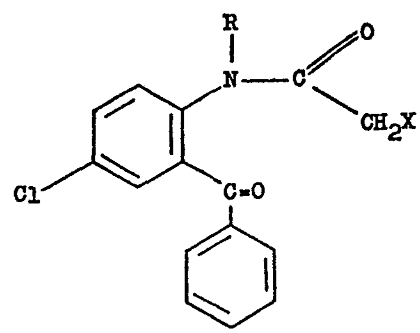

(II)

worin

R die in Anspruch 1 angegebene Bedeutung hat und

X ein Halogenatom darstellt,

mit einem Amin der Formel III

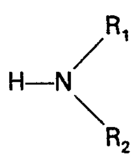

(III)

worin $R_1$ und $R_2$ die in Anspruch 1 angegebenen Bedeutungen haben, reagieren läßt.

21 Verfahren nach Anspruch 20, dadurch gekennzeichnet, daß das Halogenacetamid der Formel II hergestellt wurde durch Umsetzung eines Halogenacetylhalogenids der Formel IV

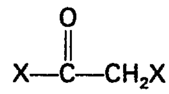

(IV)

mit einem N-substituierten Derivat von 2 - Amino - 5 - chlor - benzophenon der Formel V

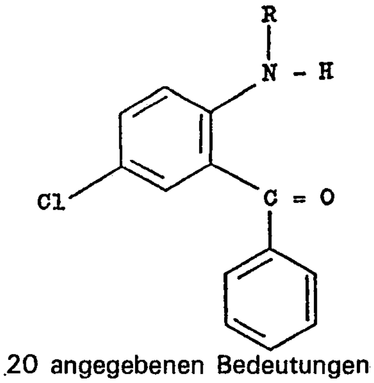

(V)

worin R und X die in Anspruch 20 angegebenen Bedeutungen haben.

22. Verfahren nach Anspruch 21, dadurch gekennzeichnet, daß das N-substituierte Derivat des 2-Amino-5-chlor-benzophenons der Formel V hergestellt wurde durch Hydrolyse einer N-substituierten Verbindung von 2-Tosylamido-5-chlor-benzophenon der Formel VI

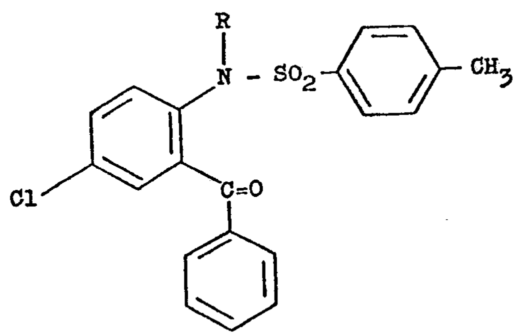

(VI)

worin R die in Anspruch 20 angegeben Bedeutungen hat.

23. Verfahren nach Anspruch 22, dadurch gekennzeichnet, daß die N-substituierte Verbindung des 2-Tosylamido-5-chlor-benzophenons der Formel VI hergestellt wurde durch Umsetzung eines Halogenids der Formel VII

$$R—X \quad \text{(VII)}$$

mit 2-Tosylamidop-5-chlor-benzophenon,
wobei R und X die in Anspruch 20 angegebenen Bedeutungen haben.

24. Verfahren nach Anspruch 23, dadurch gekennzeichnet, daß das 2-Tosylamido-5-chlor-benzophenon hergestellt wurde durch Umsetzung eines Tosylhalogenids mit 2-Amino-5-chlor-benzophenon.

25. Verfahren nach Anspruch 21, dadurch gekennzeichnet, daß das N-substituierte Derivat des 2-Amino-5-chlor-benzophenons der Formel V hergestellt wurde durch Umsetzung eines Sulfats der Formel VIII

$$(R)_2SO_4 \quad \text{(VIII)}$$

worin R die in Anspruch 20 angegebene Bedeutung hat, mit 2-Amino-5-chlor-benzophenon in Gegenwart von Essigsäure.

26. Als neue Medikamente die Verbindungen der allgemeinen Formel I nach einem der Ansprüche 1 bis 19 sowie ihre Additionssalze mit physiologisch akzeptablen Säuren.

27. Pharmazeutische Mittel, dadurch gekennzeichnet, daß sie als aktive Substanze mindestens eine Verbindung der allgemeinen Formel I nach einem der Ansprüche 1 bis 19 oder eines ihrer Additionssalze mit physiologisch akzeptablen Säuren enthalten.